# EUROPÄISCHE PATENTSCHRIFT

(11) **EP 0 685 425 B1**
(45) Veröffentlichungstag und Bekanntmachung des Hinweises auf die Patenterteilung: **29.12.1997**
(21) Anmeldenummer: 95107714.8
(22) Anmeldetag: 20.05.1995
(51) Int. Cl.: C01B 6/04

(54) **Verfahren zur Herstellung von Magnesium-hydrid**
Process for the preparation of magnesium hydride
Procédé pour la préparation d'hydrure de magnésium

(30) Priorität: 03.06.1994 DE 4419456
(43) Veröffentlichungstag der Anmeldung: 06.12.1995
(73) Patentinhaber: Th. Goldschmidt AG, 45127 Essen (DE)
(72) Erfinder: Knott, Wilfried, Dr., D-45141 Essen (DE); Rupilius, Wolfgang, Dr., D-45259 Essen (DE)

(56) Entgegenhaltungen:
- EP-A- 0 112 548
- EP-A- 0 147 581
- EP-A- 0 490 156
- CHEMICAL ABSTRACTS, vol. 114, no. 18, 6.Mai 1991 Columbus, Ohio, US; abstract no. 167809e, ANTONOVA M.M.

## Beschreibung

Die Erfindung betrifft ein Verfahren zur Herstellung von Magnesiumhydrid, welches insbesondere in aktiven, reversibel H₂ aufnehmenden Magnesiumhydrid-Magnesium-Wasserstoff-Speichersystemen eingesetzt werden kann.

Von den bekannten Metallhydrid-Metall-Systemen, die als reversible Wasserstoff-Speichersysteme in Frage kommen, zeichnet sich das MgH₂-Mg-System durch den höchsten Gewichtsanteil an reversibel gebundenem Wasserstoff (7,65 Gew.-%) und damit die höchste Energiedichte je Gewichtseinheit des Speichermaterials aus (2332 Wh/kg).

Einer Anwendung solcher Speichersysteme für mobile Zwecke stand bisher die unbefriedigende Kinetik dieses Systems im Wege. So ist es bekannt, daß sich reines Magnesium nur unter drastischen Bedingungen und auch dann nur sehr langsam und unvollständig hydrieren läßt.

Man hat deshalb versucht, die Hydrierbarkeit des Magnesiums durch Dotierung oder Legierung des Magnesiums mit Fremdmetallen, wie Al, In, Fe, Mg₂Ni, Mg₂Cu oder LaNi₅, zu verbessern. Hierdurch konnten zwar Verbesserungen hinsichtlich der Kinetik erzielt werden, jedoch blieben noch wesentliche Nachteile bestehen, wie z. B. die Notwendigkeit drastischer Bedingungen bei der Ersthydrierung des dotierten Magnesiums. Außerdem lagen die Speicherkapazitäten solcher Systeme unter dem theoretisch zu erwartenden Wert.

Von diesem Stand der Technik ausgehend befaßt sich die EP-PS 0 112 548 mit dem Problem der Verbesserung der Magnesiumhydrid-Magnesium-Wasserstoff-Speichersysteme und insbesondere mit der verbesserten Dotierung. Die EP-PS 0 112 548 lehrt ein Verfahren der oben bezeichneten Gattung mit dem Kennzeichen, daß man Magnesiumhydrid oder metallisches Magnesium in fein verteilter Form durch Kontakt mit einer Lösung eines Metallkomplexes und/oder einer metallorganischen Verbindung eines Metalls der IV. bis VIII. Nebengruppe des periodischen Systems, gegebenenfalls in Gegenwart von Wasserstoff, umsetzt, wobei das jeweilige Übergangsmetall an der Oberfläche der Magnesiumhydrid und/oder Magnesiumpartikel abgeschieden wird.

Ein Nachteil dieser Verfahrensweise ist jedoch die Notwendigkeit des Einsatzes von Metallkomplexen oder metallorganischen Verbindungen, die meist teuer, häufig gefährlich zu handhaben und/oder toxisch sind. Ein derartiges Verfahren kann deshalb aus wirtschaftlichen Gründen, aus Gründen der Umweltfreundlichkeit und in Hinblick auf Gefahrenmomente für das mit der Verfahrensdurchführung beauftragte Personal noch nicht völlig befriedigen.

Die DE-OS 40 39 278 lehrt ein Verfahren zur Hydrierung von Magnesium, wobei man dem zu hydrierenden feinteiligen Magnesium als Katalysator Magnesiumhydrid einer Teilchengröße von ≦ 400 um in einer Menge von mindestens 1,2 Gew.-%, bezogen auf zu hydrierendes Magnesium, zusetzt und die Hydrierung bei einer Temperatur von ≧ 250°C und einem Druck von 0,5 bis 5 MPa durchführt.

Hierdurch wird erreicht, daß das entstehende Magnesiumhydrid als feinteiliges, fließfähiges und gut handhabbares Produkt erhalten wird. Durch das Rühren wird auch die Hydrierungsreaktion selbst begünstigt.

Grundlage dieses Verfahrens ist die Beobachtung, daß ein dem zu hydrierenden Magnesium zugesetztes, feinteiliges Magnesiumhydrid einen autokatalytischen Effekt ausübt.

Überraschenderweise wurde nun gefunden, daß ein Zusatz von feinverteiltem, hochreaktiven Magnesium zu gewöhnlichem Magnesiumpulver dessen Hydrierbarkeit in vorteilhafter Weise gestattet. Mit anderen Worten induziert eine katalytische Menge von zugesetztem feinverteilten, hochreaktiven Magnesium die einfache Hydrierung eines kommerziellen Magnesiums.

Gegenstand der Erfindung ist somit ein Verfahren zur Herstellung von Magnesiumhydrid unter Zusatz eines Katalysators für die Wasserstoffaufnahme unter Hydridbildung, dadurch gekennzeichnet, daß man dem zu hydrierenden feinteiligen Magnesium als Katalysator feinverteiltes, hochreaktives Magnesium zusetzt und die Hydrierung bei einer Temperatur von ≧ 250°C und einem Druck von 0,5 bis 5 MPa durchführt, wobei man vorteilhaft das Reaktionsgut während der Hydrierung unter ständigem Rühren durchmischt.

Hierbei sind Mengen von 1 bis 10, vorzugsweise 2 bis 5 Gew.-%, bezogen auf das zu hydrierende Magnesium, ausreichend.

Das hochreaktive Magnesium kann dabei gegebenenfalls in situ erzeugt werden.

Der Begriff feinverteiltes, hochreaktives Magnesium umfaßt aktivierte Formen metallischen Magnesiums, die gemäß dem bekannten Stand der Technik nach zahlreichen Verfahren herstellbar sind, beispielsweise gemäß DE-OS 33 40 492 aus Magnesiumhydrid, Magnesiumanthracen und/oder seinen Derivaten oder Magnesiumbutadien und/oder seinen Alkyl- oder Phenylderivaten durch thermische Zersetzung. Die Lehre der DE-OS 33 40 492 stellt u. a. darauf ab, daß diese aktivierten Formen metallischen Magnesiums zur reversiblen Herstellung aktiven Magnesiumhydrids unter moderaten Druck- und Temperaturbedingungen geeignet sind. Dieses hochreaktive Magnesium unterscheidet sich besonders im Hinblick auf die anzulegenden Hydrierbedingungen völlig von dem handelsüblichen Magnesium, so daß eine Wasserstoffaufnahme bereits bei 150°C unter Normaldruck referiert wird.

Als eine Modifizierung der thermischen Zersetzung von Magnesiumanthracen zu hochreaktivem Magnesium wird von J. Treber (Dissertation, Kaiserslautern (1989)) auf die Möglichkeit zur Zersetzung mit Hilfe des Ultraschalles hingewiesen.

K. J. Klabunde et al. (J. Organomet. Chem. 71, 309-13 (1974)) beschreiben die Gewinnung hochaktiven Magnesiums durch Atomverdampfung.

R. C. Fuson (J. Am. Chem. Soc. 79, 928 (1957)) belegt die Möglichkeit, ein reaktives Magnesium durch intensives Aufmahlen zu generieren.

Nach R. D. Rieke kann Magnesium in einer aktiven Form durch Reduktion von Magnesiumhalogeniden mit Alkalimetallen, insbesondere mit Kalium, in Tetrahydrofuran (THF) (Acc. Chem. Res. 10, 301 (1977)) oder 1,2-Dimethoxyethan, gegebenenfalls unter Zusatz von Naphthalin als Elektronenüberträger (Arnold & Kullenovi'c, Synth. Commun. 7, 223 (1977); Rieke et al., J. Org. Chem. 46, 4323 (1981)) gewonnen werden.

Die Möglichkeit zur einfachen Hydrierung hochreaktiver, feinverteilter Formen metallischen Magnesiums ist Stand der Technik. Umso verblüffender ist die Beobachtung, daß eine katalytische Menge zugesetzten hochreaktiven Magnesiummetalls die technisch einfache Hydrierbarkeit handelsüblichen Magnesiumpulvers ermöglicht.

Eine bevorzugte Form des hier beschriebenen erfindungsgemäßen Verfahrens liegt darin, die Hydrierung von Magnesium dadurch zu initiieren, daß man ein aktives Magnesiummetall in katalytischer Menge hinzusetzt, welches zuvor durch Dehydrieren von Magnesiumhydrid gewonnen wurde, wobei die Genese dieses Magnesiumhydrids von untergeordneter Bedeutung ist.

Das Verfahren erfüllt die vorgenannten Bedingungen: es ist wirtschaftlich, umweltfreundlich und vermeidet das Arbeiten mit größeren Mengen metallorganischer Komplexe oder Verbindungen.

Obwohl das erfindungsgemäße Verfahren auf die Verwendung einer kleinen Menge zum Teil hochpyrophoren Magnesiummetalls als Katalysator zur Hydrierung handelsüblichen Magnesiums zurückgreift, ist das erhaltene feinteilige Magnesiumhydrid ein gut handhabbares, nicht-pyrophores Pulver.

Das erfindungsgemäße Verfahren wird anhand des folgenden Beispiels noch näher erläutert:

### Beispiel

2845 g (117 Mol) Magnesiumpulver (Aluma, Grade 5) mit einer mittleren Partikelgröße < 75 µm werden in einem 16-l-Druckreaktor, der zur Erfassung der wesentlichen Hydrierparameter an einen Mehrkanalschreiber angeschlossen ist, unter Argonbedampfung vorgelegt. 56,9 g (2,3 Mol) eines hochaktiven, pyrophoren Magnesiumpulvers, hergestellt nach dem Verfahren der DE-OS 33 40 492, werden unter strenger Einhaltung von Inertgasbedingungen hinzugegeben. Der Reaktor wird verschlossen und mit Hilfe einer ölpumpe bis auf einen Druck von 133 Pa evakuiert. Dann wird der Druckbehälter mit 4,0 bar Wasserstoffdruck beaufschlagt, verschlossen und unter ständigem Rühren des Festbettes auf 350°C erhitzt. 5 1/2 Stunden nach Beginn des Aufheizens sinkt der thermisch aufgebaute Maximaldruck von 9,8 bar langsam ab und ist nach einer weiteren Stunde auf 3,3 bar gefallen. An den Reaktor wird ein konstanter Druck von 10 bar Wasserstoff angelegt, was dazu führt, daß die Innentemperatur merklich steigt. Die Hydrierkinetik läßt sich über die Messung des Druckabfalls pro Zeiteinheit verfolgen, wobei Wasserstoffaufnahmen von 1,7 bar/Minute erreicht werden. Mit ausklingender Reaktion sinken sowohl die Hydriergeschwindigkeit als auch hiermit einhergehend die beobachtete Wärmetönung. Man läßt über Nacht nachrühren. Nach Erkalten wird der Reaktor entspannt und über den Bodenablaß werden 3088 g eines freifließenden, nicht-pyrophoren Magnesiumhydrids isoliert.

Der Hydridgehalt einer Probe wird nach Zh. Neorgh. Khim. 6, 1961, gasvolumetrisch durch Zersetzen mit H₂CrO₄ bestimmt. Es wird ein Hydridgehalt von 6,95 % ermittelt.

## Patentansprüche

1. Verfahren zur Herstellung von Magnesiumhydrid unter Zusatz eines Katalysators für die Wasserstoffaufnahme unter Hydridbildung, dadurch gekennzeichnet, daß man dem zu hydrierenden feinteiligen Magnesium als Katalysator feinverteiltes, hochreaktives Magnesium zusetzt und die Hydrierung bei einer Temperatur von ≧ 250°C und einem Druck von 0,5 bis 5 MPa durchführt.

2. Verfahren gemäß Anspruch 1, dadurch gekennzeichnet, daß man das Reaktionsgut während der Hydrierung unter ständigem Rühren durchmischt.

## Claims

1. Process for the preparation of magnesium hydride, with addition of a catalyst for hydrogen absorption with hydride formation, characterized in that finely divided, highly reactive magnesium is added as catalyst to the finely divided magnesium to be hydrogenated, and the hydrogenation is carried out at a temperature of ≥ 250°C and a pressure of from 0.5 to 5 MPa.

2. Process according to Claim 1, characterized in that the reaction mixture is mixed with constant stirring during the hydrogenation.

## Revendications

1. Procédé de préparation d'hydrure de magnésium par addition d'un catalyseur pour l'adsorption d'hydrogène par formation d'hydrure, caractérisé en ce que l'on ajoute comme catalyseur, au magnésium en fines particules à hydrogéner, du magnésium hautement réactif, finement divisé et en ce que l'hydrogénation est réalisée à une température de ≥250°C et sous une pression de 0,5 à 5 MPa.

2. Procédé suivant la revendication 1, caractérisé en ce que l'on mélange intimement les réactifs pendant l'hydrogénation par une agitation continue.
